(19) **Europäisches Patentamt / European Patent Office / Office européen des brevets**

(11) **EP 2 594 281 B1**

(12) # EUROPEAN PATENT SPECIFICATION

(45) Date of publication and mention
of the grant of the patent:
**28.08.2019 Bulletin 2019/35**

(51) Int Cl.:
*A61K 38/28* (2006.01)          *A61K 9/107* (2006.01)
*A61K 9/127* (2006.01)          *A61P 3/10* (2006.01)
*C07K 14/62* (2006.01)

(21) Application number: **11806298.3**

(22) Date of filing: **14.07.2011**

(86) International application number:
**PCT/CN2011/077152**

(87) International publication number:
**WO 2012/006956 (19.01.2012 Gazette 2012/03)**

(54) **INSULIN-LIPID COMPLEX, PREPARATION METHOD THEREFOR, AND PREPARATION THEREOF**

INSULIN-LIPID-KOMPLEX, HERSTELLUNGSVERFAHREN DAFÜR UND PRÄPARAT DARAUS

COMPLEXE INSULINE-LIPIDE, SON PROCÉDÉ DE PRÉPARATION ET SA PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2010 CN 201010226102**

(43) Date of publication of application:
**22.05.2013 Bulletin 2013/21**

(73) Proprietor: **INSTITUTE OF MATERIA MEDICA, CHINESE ACADEMY OF MEDICAL SCIENCES**
**Beijing 100050 (CN)**

(72) Inventors:
- **LIU, Yuling**
  **Beijing 100050 (CN)**
- **ZHOU, Cuiping**
  **Beijing 100050 (CN)**
- **SONG, Zhihui**
  **Beijing 100050 (CN)**
- **LI, Lin**
  **Beijing 100050 (CN)**
- **WANG, Hongliang**
  **Beijing 100050 (CN)**
- **XIA, Xuejun**
  **Beijing 100050 (CN)**
- **WANG, Renyun**
  **Beijing 100050 (CN)**
- **DONG, Wujun**
  **Beijing 100050 (CN)**

- **JIN, Dujia**
  **Beijing 100050 (CN)**

(74) Representative: **Hutter, Anton et al**
**Venner Shipley LLP**
**200 Aldersgate**
**London EC1A 4HD (GB)**

(56) References cited:
**WO-A1-90/11780          WO-A1-2010/060667**
**CN-A- 101 524 349          DD-A1- 212 185**
**DE-A1- 3 240 177**

- **AJAY SEMALTY ET AL: "Pharmacosomes: the lipid-based new drug delivery system", EXPERT OPINION ON DRUG DELIVERY,, vol. 6, no. 6, 1 June 2009 (2009-06-01), pages 599-612, XP009169637,**
- **LU Y ET AL: "Formulation of an intravenous emulsion loaded with a clarithromycin-phospholipid complex and its pharmacokinetics in rats", INTERNATIONAL JOURNAL OF PHARMACEUTICS, ELSEVIER BV, NL, vol. 366, no. 1-2, 21 January 2009 (2009-01-21), pages 160-169, XP025839913, ISSN: 0378-5173, DOI: 10.1016/J.IJPHARM.2008.09.008 [retrieved on 2008-09-16]**
- **PERRY M C ET AL: "THE INTERACTION OF INSULIN WITH PHOSPHO LIPIDS", BIOCHEMICAL JOURNAL, vol. 125, no. 1, 1971, pages 179-187, XP002718597, ISSN: 0264-6021**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ZHOU CUIPING ET AL: "The preparation of a complex of insulin-phospholipids and their interaction mechanism", JOURNAL OF PEPTIDE SCIENCE, vol. 18, no. 9, September 2012 (2012-09), pages 541-548, XP002718598,
- CUI, F. ET AL.: 'Biodegradable nanoparticles loaded with insulin-phospholipid complex for oral delivery: Preparation, in vitro characterization and in vivo evaluation' vol. 114, no. 2, 24 May 2006, pages 242 - 250, XP024957595
- FRICKER, G. ET AL.: 'Phospholipids and Lipid-Based Formulations' ORAL DRUG DELIVERY vol. 27, no. 8, 22 April 2010, pages 1469 - 1486, XP019827972

## Description

## Technical Field

The present disclosure relates to an insulin-lipid complex and process for preparation thereof, as well as an oil solution comprising said insulin-lipid complex and use thereof in the manufacture of a sustained-released injection and non-injection formulation. The present disclosure further relates to a new vesicle (liposome) comprising said insulin-lipid complex and use thereof in the manufacture of non-injection formulation. The present disclosure belongs to the technical field of pharmaceutical formulation.

## Background Art

### 1. Introduction of the research progress and background of lipid complexes

### 1.1 Structure characteristics and formation mechanism of the complexes

Phospholipid complexes were found by an Italian scholar Bombardelli in the study of the liposome. Early research on phospholipid complexes relates to drugs that are mostly phenolic hydroxyl-comprising flavonoids or polyphenols. Later researches prove that, in addition to phenolic hydroxyl, some polar groups such as alcoholic hydroxyl groups, amide groups or carbonyl groups might react with the hydrophilic end of phospholipid or other lipid material (such as cholesterol, sodium cholate, etc.) to form a complex spheroid by intermolecular hydrogen bonding or Van der Waals' force. Both hydrophilic drugs and lipophilic drugs can form lipid complexes as long as they contain polar groups capable of complexation. Formation of lipid complex can significantly improve the lipophilicity and oil solubility of drugs.

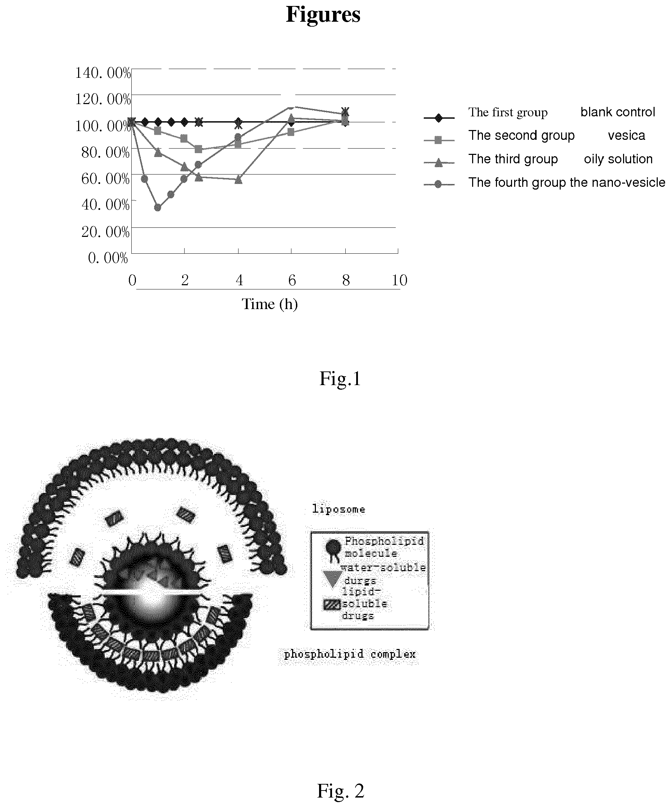

As shown in Fig. 2, the composition and structure of phospholipid complexes are markedly different from the vesicle (also known as liposome), which is also composed of phospholipids. A liposome is a vesicle structure enclosed by a bilayer membrane formed with hydrophilic ends of phospholipid molecules orientated outwards and hydrophobic ends orientated inwards, while a phospholipid complex is a lipophilic spheroid, with the polar groups of active ingredients being fixed by intermolecular interaction with the hydrophilic ends of phospholipid, so as to encapsulate the hydrophilic portion of the phospholipid, while the hydrophobic ends not participating in the complexation reaction and being free to move.

A liposome vesicle may be enclosed by hundreds or thousands of phospholipid molecules, with the outer and inner layer of the bilayer membrane being hydrophilic ends of phospholipid, while the interlayer being hydrophobic ends. Lipophilic drugs can be entrapped in the interlayer of the bilayer membrane (blue square in the Figure), with the entrapment efficiency being high and stable, and it is not easy to leak, while hydrophilic drugs can only disperse in the kernel or periphery of the vesicles enclosed by the bilayer membrane. Since it is difficult for drugs to enter into the kernel, actually the hydrophilic drugs mostly distribute in the periphery, with poor stability and being easy to leak. For mucous membrane permeability, liposome of lipophilic drug is always obviously superior to liposome of hydrophilic drug.

So, for hydrophilic drugs, a lipid complex can be firstly prepared to improve lipophilicity, and then a vesicle can be prepared to improve entrapment efficiency and stability, thus improving the transport property of mucous membrane.

### 2. Research background of insulin-lipid complexes and defects in existing technique

### 2.1 Research backgrounds of insulin-lipid complexes

Insulin is susceptible to gastric acid and various proteolytic enzymes in digestive tract and is difficult to penetrate gastrointestinal mucosal barrier due to its high molecular weight. Conventional oral formulation is invalid, and subcutaneous injection is still the main route of administration. However, patient compliance is poor with long-term frequent injection. To overcome the patient compliance problem caused by frequent injection, the domestic and foreign medical workers have carried out a large number of researches in the past few decades, by structural modifications on the one hand to prepare medium-acting or long-acting insulin, prolong drug effect duration and reduce injection frequency, and on the other hand, by preparing liposomes, nanoparticles, microspheres, micro emulsion or oil solution through technologies in pharmaceutics, to improve drug stability against acids, bases and bio-enzymes, to promote transportation and absorption of drugs through epithelium mucosa, and to provide drug release carriers for the development of non-injection insulin formulation for oral, percutaneous, mucosal, and lung inhalation administrations.

The poor lipophilicity of insulin limits the preparation and development of microparticle carriers. Insulin liposome is the most reported particle carrier both at home and abroad. However, due to the high molecular weight and strong hydrophilicity of insulin, the drug mostly exists in periphery of the phospholipid bilayer membrane, resulting in low entrapment efficiency and being easy to leak. Thus, the improvement of insulin stability in the gastrointestinal tract and mucous membrane permeability is limited. The preparations of nanoparticles and microspheres are mostly conducted in organic solvent system. But insulin is poorly soluble in organic solvent, so that the entrapment efficiency is extremely low, the insulin is only adsorbed on the particle surface, burst release of the drug readily happens after administration, and the stabilizing effect is poor as well. In the case of microemulsion or self-microemulsion reported in the existing literature, insulin is dissolved in water phase so that the drug cannot be prevent from contacting with gastric acid and bio-enzyme, which is disadvantageous for the improvement of drug stability in the gastrointestinal environment.

Insulin molecular contains a large number of polar groups, such as acylamino, phenolic hydroxyl, hydroxyl, carbonyl groups, which are capable of reacting with hydrophilic ends of lipid material by intermolecular interaction to form lipid complex, so as to improve its lipophilicity, and break through the limitation of the preparation of microparticle carriers. Researches on insulin-lipid complex become the focus of attention at home and abroad in recent years. But the poor lipophilicity of insulin also limits the preparation of lipid complex. Defects such as low complexation rate and unstable quality were widely found in the domestic and foreign literatures and patent documents.

For instance, WO 90/11780 is concerned with liposome and lipidic particle formulation of compounds. These are prepared by dissolving the components in an aprotic solvent such as DMSO and injecting the resulting solution into an aqueous solution.

Cui et al. (Journal of Controlled Release, col. 114, no. 2, pages 242-250) discloses inculin-lipid complexes which were prepared by a reverse micelle-solvent evaporation method.

DE3240177 is concerned with a physically stabilized insulin solution and a method for producing the said solution. The solution is stabilized due to containing a stabilizing amount of a phospholipid.

Semalty et al. (Expert Opinion on Drug Delivery, col. 6, no. 6, pages 599-612) reviews the potential of pharmacosomes as a controlled and targeted drug delivery system and highlights the methods of preparation and characterization.

Perry et al. (Biochemical Journal, vol. 125, no. 1, 1971, pages 179-187) uses a two-phase chloroform-aqueous buffer system to investigate the interaction of insulin with phospholipids and other amphipathic substances.

Zhou et al. (Journal of Peptide Science, vol. 18, no. 9, pages 541-548) is concerned with a technique of fabricating insulin-phospholipid complexes using a solvent evaporation method with the aim of improving the lipophilicity of insulin.

Finally, WO 2010/060667 A1 is concerned with a water-free liquid or semisold pharmaceutical composition comprising a derivatized insulin peptide, a polar organic solvent and a lipophilic component.

## 2.2 The defects of existing technique

Insulin is composed of two peptide chains A and B and has a molecular weight close to 6000, which is between polypeptide and protein. A-chain of human insulin (Insulin Human) has 21 amino acids of 11 kinds, and B-chain has 30 amino acids of 15 kinds, with a total of 51 amino acids of 26 kinds. Insulin is insoluble in water and organic solvents, but soluble in diluted acidic alcohol, phosphate buffer with pH 7.4, diluted acid and diluted base.

Insulin contains a lot of polar groups capable of reacting with hydrophilic ends of lipid material by intermolecular interaction, and meets the requirement of formation of lipid complex. However, due to the protein structure characteristics and physical and chemical properties of insulin, the preparation of lipid complex is extremely difficult, with the biggest obstacle being the choice of complexation solvents.

Organic solvent, especially aprotic solvent is advantageous for the complexation reaction. However, insulin is insoluble in organic solvent, and organic solvent may cause degradation or conformational change of insulin. Therefore, it is impossible to merely use organic solvent to prepare insulin complex, and so far it has not been reported in the literature. If choosing phosphate buffer with pH7.4, in which insulin has certain solubility and stable quality, lipid material is not able to dissolve to form a clear and transparent solution. In addition, due to the high polarity of water, the entrapment efficiency of the resulting complex is extremely low, and the quality is unstable due to weak intermolecular forces. Wo Weihan [Wo Weihan, recombinant human insulin complex, and its preparation methods and drug composition containing the complex, Chinese patent: 01140047, 2004-10-06] discloses using phosphate buffer as solvent, dissolving or suspending insulin and phospholipid in aqueous solution, mixing homogeneously, and removing water by freeze drying to prepare phospholipid complexes. The results showed that, when the mass ratio of phospholipid to drug is 25:1 (mole ratio of about 185:1), the complexation rate is only 21.35%, when the mass ratio is 150:1 (mole ratio of about 1110:1), the complexation rate is 72.0%.

Theoretically, insulin contains 53 acylamino groups, 4 phenolic hydroxyl groups, 12 alcoholic hydroxyl groups, which are all likely to complex with phospholipid. That is to say, about 70 mole phospholipid is needed for 1 mole drug in theory (weight ratio of about 1:10). Usually, in order to ensure complete complexation of the drug, the inventory of lipid material should be slightly higher than the theoretical value. When calculated by 1.5 times of theoretical value, the maximum amount of lipid material shall not exceed 15 times of the drug by mass. That is, the amount of phospholipid should be controlled no more than 15 times that of insulin by mass, which is economic and reasonable. But in No.

01140047 patent, a complete complexation is not achieved under a phospholipid mass of 150 times as high as that of insulin, indicating that the complexation efficiency in water solvent is too low.

Another disclosure [R·R·C·New, Hydrophobic formulation containing medium chain monoglyceride, Chinese patent: 97196069, 1999-07-28] called as Macrosol technique, provides an insulin oil solution containing lipid complex. In the preparation of said lipid complex, drug and amphipathic lipid material are dissolved in aqueous solvent of a buffer salt, followed by the solvent being removed by rotary evaporation or freezing drying, and then the resultant being dissolved in oil phase to give an oil solution (or directly mixing the complexing solution with oil, followed by freeze drying). The determined weight ratio of insulin to phospholipid is 1:1 to 1:20, preferably 1:2 to 1:8, without providing the evaluation result of the complexation rate. In view of the fact disclosed in NO.01140047 patent that it is difficult to achieve a complex of high complexation rate using water as solvent, it is readily speculated that, for the complex prepared under the condition disclosed in No. 97196069 patent, most of the drug may not have complexed with the phospholipid.

The present inventors verified the method of No.97196069 patent. According to the fact that insulin complex is soluble in cyclohexane while free insulin is not, a complexation rate determination method (HPLC quantitative methods) is established. Insulin phospholipid complexes are prepared according to the method disclosed in No. 97196069 patent. When the mass ratio of insulin/phospholipid is 1:2, the complexation rate is below 8%; when the mass ratio is 1:8, the complexation rate is not in excess of 21%; and when the mass ratio is 1:12, the complexation rate is not in excess of 25%. Complexes with insulin/phospholipid mass ratio of 1:8 and 1:12 are further dissolved in medium chain oil with simultaneously stirring to prepare medium chain oil solution with a drug concentration of 1.5 mg/g. After 1 month of storage under room temperature or 3 months of storage in refrigerator (2-8 °C), turbid phenomenon occurs in both circumstance.

Although the mass ratio of insulin/phospholipid determined in No. 97196069 patent is close to the theoretical value, the complexation rate is extremely low due to the using of water as complexation solvent in its preparation, this is in consistent with the results of No. 01140047 patent.

It can be seen from the above two patents, using of water as solvent in the preparation of insulin-lipid complexes results in a low complexation rate and apparent defect.

Solvent systems in some subsequent patents or reports are improved based on No. 97196069 patent, by selecting solvents such as ethanol containing glacial acetic acid, DMSO containing glacial acetic acid, or ethyl ether containing diluted aqueous hydrochloric acid solution as the complexation reaction solvent. Compared with water solution, organic solvent has weaker polarity. And the addition of acid increases the solubility of insulin and improves the complexation efficiency. A verification test was also conducted by the present inventors. The result shows that, due to strong acidity of glacial acetic acid and aqueous hydrochloric acid and their low volatility, the insulin content decreases by about 5-10% during preparation, and continues to decrease by 20% or more during storage.

In summary, with regard to preparation of insulin-lipid complexes, the existing methods disclosed in the patents and documents, due to improper choice of solvent systems, suffer from low complexation efficiency, ease of degradation during drug preparation, affecting of quality stability during storage by acid component or water residue. In addition, since the ratio of drug to lipid material is not optimized, the resulting complexes have low complexation rate so that the improvement of solubility in oil phase is limited. And the resulting oil solutions have low drug loading capacity and readily suffer from instability such as turbidity during storage.

## Summary of the Invention

The present invention provides a process for the preparation of an insulin-lipid complex, comprising:

- dissolving an insulin in methanol containing trifluoroacetic acid to form a clear insulin solution;

- dissolving a lipid material in dichloromethane to form a clear lipid solution;

- homogeneously mixing of the insulin solution and the lipid solution; and

- conducting rotary evaporation under reduced pressure in a water bath, evacuating by a water pump, and drying,

wherein the insulin is recombinant human insulin, the lipid material is a natural phospholipid or a salt thereof, the mass ratio of the insulin to the lipid material is between 1:5 and 1:10, the concentration of trifluoroacetic acid in methanol is 0.05-0.1%, and the amount of dichloromethane is 4-6 times of that of methanol.

The natural phospholipid may be selected from egg yolk phospholipid or soybean phospholipid

The disclosure also provides an insulin-lipid complex, which is complexed by an insulin and a lipid material in an organic solvent system containing a low boiling point acid. In the complex, the mass ratio of said insulin to said lipid material is 1:3~1:15; preferably 1:4~1:12; more preferably 1:5~1:10.

The insulin may be one selected from the group consisting of natural insulins, porcine insulin, bovine insulin, recombination human insulins and various medium-acting or long-acting insulins, preferably recombination human insulins. The lipid material is selected from the group consisting of natural phospholipids, synthetic phospholipids, cholesterol, cholic acid and salt thereof, or a combination thereof. The lipid material is preferably selected from natural phospholipids, preferably the natural phospholipids are selected from egg yolk phospholipid or soybean phospholipid.

An insulin-lipid complex may further contain one or more additional ingredients selected from the group consisting of antioxidant, metal chelating agent and protease inhibitors.

The complexation solvent used may be an organic solvent containing a low boiling point acid, wherein the low boiling point acid is one selected from the group consisting of trifluoroacetic acid, and hydrogen chloride gas, or a combination thereof. The organic solvent is one selected from the group consisting of methanol, tetrahydrofuran, DMSO, chloroform, dichloromethane, and ethyl ether, or a combination thereof.

The insulin-lipid complex can be prepared by the following methods:

Method 1) firstly adding a suitable amount of trifluoroacetic acid or introducing a suitable amount of hydrogen chloride gas into an organic solvent, then adding an insulin and a lipid material, stirring for both to fully complex until formation of a clear and transparent solution, removing the organic solvent by rotary evaporation or spray drying, and drying the residue.

Method 2) firstly dissolving a lipid material in an organic solvent, then adding an insulin, slowly introducing a suitable amount of hydrogen chloride gas or adding a suitable amount of trifluoroacetic acid under stirring condition until the insulin completely dissolves to form a clear and transparent solution, stirring or ultrasonic processing at room temperature for a given time to fully complex the insulin and the lipid material, removing the organic solvent by rotary evaporation or spray drying, and drying the residue.

Method 3) dissolving an insulin in solvent A containing a suitable amount of trifluoroacetic acid or hydrogen chloride gas to form a clear insulin solution, and dissolving a lipid material in a suitable amount of solvent B to form a clear lipid solution, after homogeneously mixing of the insulin solution and the lipid solution, conducting rotary evaporation under reduced pressure in a water bath, evacuating by a water pump, and drying.

Method 4) dissolving an insulin in solvent A containing a suitable amount of trifluoroacetic acid or hydrogen chloride gas to form a clear insulin solution, and dissolving a lipid material in a suitable amount of solvent B to form a clear lipid solution, after homogeneously mixing of the insulin solution and the lipid solution, conducting rotary evaporation under reduced pressure and a given temperature in a water bath, with a suitable amount of solvent B being gradually added during the rotary evaporation, evacuating the solvent by a water pump, and drying.

In the above mentioned Method 1) and Method 2), said "organic solvent" is one selected from the group consisting of methanol, tetrahydrofuran, and DMSO, or a combination thereof, preferably methanol. The adding amount of trifluoroacetic acid or the introducing amount of hydrogen chloride gas is sufficient for the added insulin to be fully dissolved. Preferably, the concentration of acid in organic solvent is 0.01-0.5%, more preferably 0.05-0.1% (weight/volume, g/ml).

In the above mentioned Method 3) and Method 4), said "solvent A" is one selected from the group consisting of methanol, tetrahydrofuran, and DMSO, or a combination thereof, preferably methanol; said "solvent B" is one selected from the group consisting of chloroform, dichloromethane, and ethyl ether, or a combination thereof, preferably dichloromethane. The concentration of trichloroacetate or hydrogen chloride gas in solvent A is about 0.01-0.5%, preferably 0.05-0.1%. The amount of solvent B is about 3-8 times, preferably 4-6 times of that of solvent A.

In the complexing solution of insulin and lipid material, the concentration of insulin should be controlled to 0.5~30mg/ml, preferably 1.0~10.0mg/ml. In the term "stirring or ultrasonic processing at room temperature for a given time", "room temperature" means 15 °C ~30 °C, for example 15 °C, 20 °C, 25 °C or 30 °C; "a given time" means within 30 min, for example 30 min, 20 min, 10 min or 5 min.

The organic solvent can be removed by rotary evaporation method, freeze drying method, and other solvent removing method as long as it removes solvent completely and has no influence on the stability of drugs. When removing the solvent with rotary evaporation method, said method should be conducted below 40 °C, and specifically 35 °C, 30 °C or 25 °C.

The present disclosure further provides an insulin oil solution formulation, containing an insulin-lipid complex, as defined above, and an oil. The oil is one selected from the group consisting of long chain triglycerides (long chain oils), medium chain triglycerides (medium chain oils), glyceryl monooleates, glyceryl monostearates, ethyl oleate, and isopropyl myristate, or a combination thereof. In the oil solution containing an insulin-lipid complex, the oil can optionally further contain one or more emulsifiers selected from the group consisting of Tween 80, Span 20, Brij, polyoxyethylene hydrogenated castor oil (Cremphor RH40), polyoxyethylene castor oil (Cremphor EL35) and Labrosal.

In the oil solution containing an insulin-lipid complex, the oil can optionally further contain one or more co-emulsifiers selected from the group consisting of propanediol, PEG400 and Transcutol P.

In the oil solution containing an insulin-lipid composite, the drug content can be 12mg/g, 10mg/g, 8mg/g, 6mg/g, 5mg/g, 4mg/g, 2mg/g or less.

The present disclosure further provides use of the insulin-lipid complex defined above in the manufacture of insulin sustained-released injection formulation. The present disclosure further provides use of the insulin-lipid complex oil solution in the manufacture of non-injection insulin formulation for oral, percutaneous, mucosal, or lung inhalation administration.

The present disclosure further provides a new insulin vesicle, containing an insulin-lipid complex, as defined above, and a phospholipid, and optionally a suitable amount of one or more mixed surfactants such as Tween20, Span60, with an average particle size of about 20nm-200 nm.

The new vesicle containing an insulin-lipid complex can be aqueous dispersion, or solid powder prepared by freeze drying or spray drying.

The present disclosure further provides use of the new vesicle containing an insulin-lipid complex in the manufacture of non-injection insulin formulation for oral, percutaneous, mucosal, or lung inhalation administration.

Compared with the prior art, the complex defined above has the following advantages:

1) organic solvent system containing a low boiling point acid is used as complexation solvent: the complexation solvent is free of water; the trifluoroacetic acid and the hydrogen chloride gas with low boiling point are easy to remove by evaporation; not only acidic environment was provided for insulin to dissolve, but also the duration of removing solvent by evaporation is shortened; the selected organic solvent can ensure the formation of clear complex solution composed of insulin and lipid material, and its polarity can ensure the complexation stability of insulin and lipid material, without affecting the quality stability of insulin; the resulting complex contains no acid material or water residue; the complexation rate is no less than 90%; and no obvious change in drug content occurs during preparation and storage.

Reasonable drug/lipid material ratio: based on the breakthrough of complexation solvent, a mass ratio of insulin to lipid material of 1:3~1:15 is sufficient to obtain a fully complexed complex. Lipid material inventory is in consistent with the theoretical value.

3) Preparation of stable oil solution: the complex significantly improves the oil solubility of insulin, and the resulting oil solution has high drug loading capacity and good stability, and no turbidity occurs during long-term storage, with its physical and chemical properties being stable.

4) Preparation of stable new vesicles: the complex significantly improves the lipophilicity of the insulin, resulting in drug distribution in the interlayer of the bilayer membrane of vesicle, and significant improvement of stability of the drugs in gastric and intestinal juice and mucous membrane transport efficacy.

In the present disclosure, unless expressly indicated otherwise, all scientific and technological terms and names used in this disclosure have the same meaning commonly understood by one skilled in the art which the disclosure belongs to. Additionally, unless expressly indicated otherwise, all materials and their contents or ratios, equipment, instruments, preparation conditions are well known for one skilled in the art or obvious from the description of the present disclosure.

**Description of figures**

Fig. 1: Curves of decrease of blood sugar by the oil solution and the new vesicle containing insulin-lipid complex.

Fig. 2: Structure diagrams of phospholipid complex and liposome.

**Preparation Examples**

**1. Early exploratory research results**

**1.1 Influence of organic solvent on chemical properties and spatial structure of drugs**

In the earlier research work, the present inventors systematically investigated different organic solvents by a specific method as follows: adding a suitable amount of methanol, ethanol, acetone, tetrahydrofuran, ethyl acetate, ethyl ether or chloroform respectively into a suitable amount of insulin solution (pH7.4PBS), fully mixing and then storing for 1 hour, drying by nitrogen flushing, adding PBS solution (pH7.4) to re-dissolve, filtering and conducting HPLC measurement, comparing with a control PBS solution with the same insulin concentration, and calculating the change of insulin content. The results showed that in methanol the drug content has no obvious change and is the most stable, and in the case of tetrahydrofuran the drug is the second most stable. The ethanol or acetone results in drug content decrease by about 5-10% and in the case of ethyl ether decrease by about 15%. While in ethyl acetate, chloroform or tetrahydrofuran, the drug content significantly decreased by about 30-40%. Those results indicate that the chemical property of insulin is relatively stable in methanol or tetrahydrofuran. In addition to that, DMSO and DMF were also investigated. Due to the high boiling points of DMSO and DMF, they are difficult to be dried by nitrogen flushing. Therefore freeze drying method is used to remove the solvent, followed by adding of PBS solution with pH 7.4 for re-dissolving. After filtering, HPLC measurement was conducted and the insulin content calculated as above. The results showed that DMF results in significant insulin content decrease. This may be relevant to its basic property. While in the case of DMSO, the quality is relatively stable.

Operation was further conducted according to the above method of treatment with methanol, tetrahydrofuran, and DMSO. After removing the solvent, the resultant is dissolved with 5 mM PBS (pH7.4) to create a test solution containing 0.1 mg/ml insulin. The test solution is placed in a quartz cuvette with optical path of 0.1 cm and assayed in the far-ultraviolet region (190 nm~250 nm) by circular dichroism spectroscopy. The characteristic peaks and minimum ellipticity of the spectra for secondary structure were recorded. Another test solution was placed in a 1 cm cuvette, and assayed in the near-ultraviolet region (250 nm~350 nm). The characteristic negative peaks and minimum ellipticity of the spectra for tertiary structure were recorded. The results showed that for insulin treated by three kinds of solvents, the spectra for secondary structure showed two negative peaks, at 210 nm and 223 nm respectively, with the minimum ellipticity being -10.63 and -8.45 respectively; while the spectra for tertiary structure had a negative peak at 274.5 nm, with the minimum ellipticity being about -2.26. Compared to the results of insulin in PBS not treated with organic solvent, no apparent differences were observed, indicating that methanol, tetrahydrofuran and DMSO do not lead to change of spatial structure.

**1.2 Influence of addition of glacial acetic acid or aqueous hydrochloric acid on the complex quality**

The existing literatures mostly propose adding glacial acetic acid or aqueous hydrochloric acid into organic solvent to dissolve insulin and to form a clear and transparent solution.

Glacial acetic acid has high boiling point, thus rotary evaporation thereof is time consuming. With the volatilization of the organic solvent, the concentration of glacial acetic acid increases, resulting in degradation or denaturation of insulin. Especially the residual glacial acetic acid in the final complex cannot be removed, resulting in lower storage stability of the complex. In the case of complex with relatively higher amount of residual glacial acetic acid, the drug content will significantly decrease even if being dissolved in an oil solution. The drug content usually decreases during the first 24 hours.

As methanol has no influence on the insulin content, the present inventors once chose methanol containing 1-5% glacial acetic acid as complexation solvent. The complex was prepared with a mass ratio of drug/phospholipid of 1:10, by solvent-removing via rotary evaporation at 35°C followed by vacuum drying for 48 hours. The resulting complex was measured and showed a complexation rate of no less than 98%. However, determined by the gas chromatographic method, the residue content of glacial acetic acid is greater than 0.5%. After storage at a temperature of 2-8°C for 4 weeks, the insulin content decreased by about 20% as compared with the initial content. After the complex being dissolved in medium chain oil and stored at room temperature for 24 hours, the content decreased by about 15% as compared with the initial content, indicating that the residue of glacial acetic acid has significant influence on the product stability.

The present inventors further tested with methanol solution containing diluted aqueous hydrochloric acid as reaction solvent by 35 °C rotary evaporation (temperature up to 50 °C will significantly affect the quality of insulin, so it should usually be controlled below 40 °C and the duration should not be too long). The results showed that, due to the introduction of water, it becomes more difficult to remove the solvent, and complex was not well formed. The residue content of hydrochloric acid is about 0.2 % determined by gas chromatographic method. After storage at a temperature of 2-8°C for 4 weeks, the insulin content in the complex decreased by about 10% as compared with the initial content.

After the complex being dissolved in medium chain oil and stored at room temperature for 24 hours, the content decreased by about 5% as compared with the initial content.

**1.3 Investigation on methanol (containing 0.1% trifluoroacetic acid)-dichloromethane as complexation solvent**

Methanol (containing 0.1% trifluoroacetic acid)-dichloromethane was used as solvent. The mass concentration of drug was set to be 1.5mg/ml, and the ratio of insulin to soybean phospholipid was set to be 1:1, 1:3, 1:5, 1:7.5, 1:10, 1:15 and 1:20 (w/w) respectively. Insulin dissolved in methanol was mixed with phospholipid dissolved in dichloromethane, followed by solvent-removing via rotary evaporation in a water bath at 37°C as well as nitrogen flushing.

The complexation rate and the solubility in oil were determined by the following methods.

► Complexation rate (entrapment efficiency): Complexation rate measurement was conducted by using the fact that insulin complex is soluble in cyclohexane, while free insulin is not.

<u>Measurement of total drug content of the complex</u>: A suitable amount of insulin phospholipid complex was accurately weighed, and dissolved and diluted to a constant volume by adding methanol containing 1% glacial acetic acid. A test solution was obtained with homogeneously mixing. A suitable amount of control insulin was accurately weighed and dissolved with PBS solution (pH 7.4) to give a solution with a concentration of 1mg / ml. A control solution was obtained by diluting with methanol containing 1% glacial acetic acid to a concentration of 0.2mg/mL. 10μL test solution and control solution were accurately measured respectively to conduct HPLC measurement, with 0.2%TFA : acetonitrile = 70 : 30 as mobile phase, column temperature of 30 °C, flow rate of 1 mL/min, and wavelength of 214 nm, using chromatographic column Agilent ZORBAX 300 SB-C8. In the measurement, the total drug content in the complex is calculated by external standard method based on the peak area, and is denoted as $W_{total}$.

<u>Measurement of content of drug bonded with phospholipid in the complex</u>: A suitable amount of insulin phospholipid complex (containing about 10mg insulin) was accurately weighed and placed in a 10 mL volumetric flask, followed by addition of cyclohexane to a constant volume and homogeneously mixing. The complexed free insulin was removed by filtration via an organic membrane with pore size of 0.45 μm. 2mL subsequent filtrate was accurately taken into a 10 mL volumetric flask, followed by solvent-removing via nitrogen flushing. Methanol containing 1 % glacial acetic acid was added to dissolve and dilute to a constant volume with homogeneously mixing. The above HPLC measurement was conducted and the drug content was calculated by external standard method, denoted as $W_{complex}$.

The Complexation rate is calculated according to the following formula:

$$\text{Complexation rate } \% = (W_{complex} / W_{total}) \times 100 \%$$

► solubility in oil: A suitable amount of insulin phospholipid complex was mixed with soybean oil or medium chain oil and stirred with magnetic stirrer at 30 °C for 6h to get fully mixed and dissolved, followed by storage at 30 °C for 24h. Whether the drug was separated out was investigated. If no drug was separated out, a suitable amount of insulin phospholipid complex was further added until the drug was separated out. Sampling was made in an amount of 5 ml, followed by filtering with filter membrane with pore size of 0.45 μm. The consequent filtrate was diluted with methanol containing 1% acetate to a suitable extent. HPLC measurement was conducted and the apparent solubility in soybean oil and medium chain oil was calculated.

The results of 7 complexes are shown below:

Influence of ratio of drug to phospholipids on the complexes

| Insulin: Phospholipid | Content (%) | Complexation rate (%) | Solubility in Medium Chain Oil (mg/g) |
|---|---|---|---|
| 1: 1 | 86.34 | 4.48 | 0.116 |
| 1: 3 | 91.23 | 9.76 | 1.492 |
| 1: 5 | 94.77 | 98.5 | 2.39 |
| 1: 7.5 | 96.17 | 97.3 | 7.83 |
| 1: 10 | 98.31 | 96.0 | 7.09 |
| 1: 15 | 98.74 | 96.6 | 6.59 |
| 1: 20 | 97.82 | 93.2 | 6.71 |

The results show that the ratio of drug to phospholipid has a significant influence on drug content, complexation rate and solubility. The drug content increases with decrease of the insulin percentage in the system. When the mass ratio of insulin to phospholipid is 1:5, the two materials complex completely, while when the ratio is above 1:15, the complexation rate has a decreasing tendency. The solubility in medium chain oil increases with increase of the phospholipid percentage in the system. When the ratio between the two materials is above 1:7.5, the solubility tends to be constant.

**1.4 Investigation on methanol (with hydrogen chloride gas introduced) as complexation solvent**

Methanol (with a suitable amount of hydrogen chloride gas introduced) was used as solvent. The mass concentration of drug was set to be 2mg/ml and the ratio of insulin to soybean phospholipid was set to be 1:1, 1:3, 1:5, 1:7.5, 1:10, 1:15 and 1:20 (w/w) respectively. Insulin and lipid material were both dissolved in methanol, followed by stirring at room temperature for 10 minutes to fully dissolve the lipid material and drug to form a clear solution, which was then transferred to a rotary evaporation flask to remove the solvent by rotary evaporation at 35 °C. Vacuum drying was conducted at room temperature and reduced pressure for over 12 hours. The complexation rate and the solubility in medium chain oil were determined according to the method described in the above subsection 3.3. The results were as follows:

Influence of ratio of drug to phospholipids on the complexes

| Insulin: Phospholipid | Content (%) | complexation rate (%) | Solubility in Medium Chain Oil (mg/g) |
| --- | --- | --- | --- |
| 1: 1 | 82.55 | 4.16 | 0.12 |
| 1: 3 | 90.12 | 8.83 | 1.45 |
| 1: 5 | 92.57 | 97.3 | 2.72 |
| 1: 7.5 | 94.68 | 98.7 | 7.32 |
| 1: 10 | 98.72 | 97.9 | 8.51 |
| 1: 15 | 97.29 | 97.5 | 7.33 |
| 1: 20 | 96.33 | 94.1 | 6.43 |

The purpose of this disclosure is to choose the appropriate complexation solvent systems, improve the complexation efficiency and quality stability of insulin and lipid material.
The selected solvent systems can simultaneously meet the following requirements:

1) Lipid material and insulin can be both dissolved to form a clear and transparent solution;

2) The system is free of water and has small polarity, which is advantageous for intermolecular complexation between insulin and lipid material;

3) The solvent system has high evaporation efficiency and is easy to be volatilized, without acidic ingredients or water residual;

4) The property of insulin is stable during preparation.

**Example 1: Preparation of insulin complex containing different ratios of soybean lecithin**

9 portions of insulin (0.2g for each portion) were individually weighed into a conical flask, followed by addition of soybean lecithin in an amount of 0.6g, 1g, 1.2g, 1.4g, 1.6g, 1.8g, 2.0g, 2.4g and 3.0g respectively and addition of 20ml methanol solution containing hydrogen chloride gas (in a concentration of 0.1%, weight/volume, g/ml). Stirring was conducted at room temperature for 10 min so that the lipid material and the drug dissolve to form a clear solution, which was then transferred to a rotary evaporation flask so as to remove the solvent at 35 °C by rotary evaporation, followed by vacuum drying at room temperature and under reduced pressure for over 12 hours. Nine complex powders with a drug/phospholipid weight ratio of 1:3~1:15 were obtained.
Gas chromatographic method was conducted for the 9 complexes. No residue of hydrogen chloride gas was observed.

**Example 2: Preparation of insulin complexes containing different ratios of egg yolk lecithin**

8 portions of insulin (0.2g for each portion) were individually weighed into a conical flask, followed by addition of egg yolk lecithin in an amount of 0.6g, 1g, 1.2g, 1.4g, 1.6g, 1.8g, 2.0g, 2.4g and 3.0g respectively and addition of 20ml methanol solution containing hydrogen chloride gas (in a concentration of 0.1%, weight/volume, g/ml). Stirring was conducted at room temperature for 10 min so that the lipid material and the drug dissolve to form a clear solution, which was then transferred to a rotary evaporation flask so as to remove the solvent at 35 °C by rotary evaporation, followed by vacuum drying at room temperature and under reduced pressure for over 12 hours. 9 complex powders with a drug/phospholipid weight ratio of 1:3~1:15 were obtained.

Gas chromatographic method was conducted for the 9 complexes. No residue of hydrogen chloride gas was observed.

**Example 3: Preparation of insulin complexes containing different ratios of soybean lecithin**

9 portions of insulin (0.2g for each portion) were individually weighed into a conical flask, followed by addition of a suitable amount of methanol (containing 0.1% v/v of trifluoroacetic acid), such that the insulin concentration was controlled to be 10mg/ml~2mg/ml. Stirring was conducted at room temperature so that the lipid material and the drug dissolve to form a clear solution, followed by addition of soybean lecithin in an amount of 0.6g, 1g, 1.2g, 1.4g, 1.6g, 1.8g, 2.0g, 2.4g and 3.0g respectively and addition of a suitable amount (about 3-6 times of the amount of methanol) of dichloromethane. Rotary evaporation was conducted at 37 °C and under reduced pressure in a water bath, during which a suitable amount (about 1-2 times of the amount of methanol) of dichloromethane was further added, followed by substantially drying and then evacuating for 10 min by water pump.

Gas chromatographic method was conducted for the 9 complexes. No residue of trifluoroacetic acid was observed.

**Example 4: Preparation of insulin complexes containing different ratios of egg yolk lecithin**

9 portions of insulin (0.2g for each portion) were individually weighed into a conical flask, followed by addition of a suitable amount of methanol (containing 0.1% v/v of trifluoroacetic acid), such that the insulin concentration was controlled to be 10mg/ml~2mg/ml. Stirring was conducted at room temperature so that the lipid material and the drug dissolve to form a clear solution, followed by addition of egg yolk lecithin in an amount of 0.6g, 1g, 1.2g, 1.4g, 1.6g, 1.8g, 2.0g, 2.4g and 3.0g respectively and addition of a suitable amount (about 3-6 times of the amount of methanol) of dichloromethane. Rotary evaporation was conducted at 37 °C and under reduced pressure in a water bath, during which a suitable amount (about 1-2 times of the amount of methanol) of dichloromethane was further added, followed by substantially drying and then evacuating for 10 min by water pump.

Gas chromatographic method was conducted for the 9 complexes. No residue of trifluoroacetic acid was observed.

**Example 5: Preparation of insulin complexes containing different ratios of sodium deoxycholate**

8 portions of insulin (0.2g for each portion) were individually weighed into a conical flask, followed by addition of sodium deoxycholate in an amount of 1g, 1.2g, 1.4g, 1.6g, 1.8g, 2.0g, 2.4g and 3.0g respectively and addition of 20ml tetrahydrofuran solution containing hydrogen chloride gas (in a concentration of 0.1%, weight/volume, g/ml). Stirring was conducted at room temperature for 5 min. The resultant was then transferred to a rotary evaporation flask so as to remove the solvent at 35 °C by rotary evaporation, followed by vacuum drying at room temperature and under reduced pressure for over 12 hours. 8 complex powders with a ratio of 1:5~1:15 were obtained.

Gas chromatographic method was conducted for the 8 complexes. No residue of hydrogen chloride gas was observed.

**Example 6: Preparation of insulin complexes containing different ratios of sodium deoxycholate**

9 portions of insulin (0.2g for each portion) were individually weighed into a conical flask, followed by addition of a suitable amount of methanol (containing 0.1% v/v of trifluoroacetic acid), such that the insulin concentration was controlled to be 10mg/ml~2mg/ml. Stirring was conducted at room temperature so that the lipid material and the drug dissolve to form a clear solution, followed by addition of sodium deoxycholate in an amount of 0.6g, 1g, 1.2g, 1.4g, 1.6g, 1.8g, 2.0g, 2.4g and 3.0g respectively and addition of a suitable amount (about 3-6 times of the amount of methanol) of dichloromethane. Rotary evaporation was conducted at 37 °C and under reduced pressure in a water bath, during which a suitable amount (about 1-2 times of the amount of methanol) of dichloromethane was further added, followed by substantially drying and then evacuating for 10 min by water pump.

Gas chromatographic method was conducted for the 9 complexes. No residue of hydrogen chloride gas was observed.

**Example 7: Preparation of insulin-lipid complexes using DMSO instead of methanol**

3 portions of insulin (0.2g for each portion) were individually weighed into a conical flask, followed by addition of soy phosphatidylcholine, egg yolk phosphatidylcholine and sodium deoxycholate each in an amount of 2.0g and addition of 15ml DMSO solution containing hydrogen chloride gas (in a concentration of 0.1%, weight/volume, g/ml). Stirring was conducted at room temperature for 15 min for reaction. After pre-freezing below -40 °C, the solvent was removed by freeze drying. 3 complex powders were obtained.

Gas chromatographic method was conducted for the 3 complexes. No residue of hydrogen chloride gas was observed.

**Example 8: Oil solutions containing insulin/phospholipid complex**

1.8g soy phosphatidylcholine was added into 30ml methanol, followed by stirring to dissolve and addition of 0.2g insulin. Hydrogen chloride gas was introduced to form a clear solution, which was then stirred at room temperature for 5 min and subjected to rotary evaporation at 35°C to remove solvent, followed by vacuum drying at room temperature and under reduced pressure for over 12 hours to obtain a complex.

5 portions of complex (0.3g for each portion) were individually weighed, followed by addition of glyceryl monooleate, medium chain Triglycerides (medium chain oil), ethyl oleate and isopropyl myristate respectively in an amount of 2.7g. Stirring was conducted to dissolve the complex, obtaining oil solutions with a drug loading capacity of 10mg/g, followed by filtering.

After 24 hours of rest at room temperature, the above mentioned oil solutions kept clear. According to the HPLC measurement, the residue contents were 99.7% of the initial value, indicating that no degradation occurred to the drug. After 6 months storage at temperature of 2-8°C, the above mentioned oil solutions kept clear. According to the HPLC measurement, the residue contents were 99.1% of the initial value, indicating a stable quality.

**Example 9: Oil solutions containing insulin/phospholipid complexes**

2 portions of each of the complexes obtained from examples 1 to 4 (each with a drug/lipid ratio of 1:10, w/w) were individually weighed, followed by addition of medium chain triglyceride (medium chain oil) or long chain triglyceride (long chain oil) to sum up 10g respectively. Stirring was conducted to dissolve the complexes, obtaining oil solutions with a drug load of 1mg/g, 2mg/g, 3mg/g and 5mg/g.

| Formulation Composition | Formulation 1 | Formulation 2 | Formulation 3 | Formulation 4 |
|---|---|---|---|---|
| Complex | Example 1 | Example 2 | Example 3 | Example 4 |
| Complex Amount | 110mg | 220mg | 330mg | 550mg |
| Drug Load | 1mg/g | 2mg/g | 3mg/g | 5mg/g |

Medium chain oil and long chain oil were respectively added to sum up 10g so as to prepare 8 samples.

After 24 hours of storage at room temperature, the above mentioned oil solutions kept clear. According to the HPLC measurement, the residue contents were all no less than 98.5% of the initial value, indicating that no degradation occurred to the drugs. After 6 months of storage at temperature of 2-4°C, the above mentioned oil solutions kept clear. According to the HPLC measurement, the residue contents were all no less than 99.4% of the initial value, indicating a stable quality.

**Example 10: Oil solutions containing insulin/phospholipid complexes (containing emulsifier)**

3 portions of medium chain triglyceride (medium chain oil) (lOg for each portion) were weighed, followed by the addition of Tween 80 in an amount of 1g, 2g and 4g respectively. After homogeneously mixing, oil phases containing emulsifier were prepared.

| Formulation Composition | Oil Phase 1 | Oil Phase 2 | Oil Phase 3 |
|---|---|---|---|
| Medium Chain Triglyceride | 10g | 10g | 10g |
| Tween 80 | 1g | 2g | 4g |

3 portions of complexes obtained from examples 5 to 7 respectively (each with a drug/lipid ratio of 1:10, w/w) were individually weighed in a suitable amount, followed by addition of the above mentioned 3 oil phases with different emulsifier ratio respectively, each in an amount of 9.45g. Stirring was conducted to dissolve, obtaining oil solutions with a drug load of 5mg/g, followed by filtering.

| Formulation Composition | Formulation 1 | Formulation 2 | Formulation 3 |
|---|---|---|---|
| Complex in an Amount of 550mg | Example 5 | Example 6 | Example 7 |
| Oil phase in an Amount of 9.45g | Oil Phase 1 | Oil Phase 2 | Oil Phase 3 |

After 24 hours of storage at room temperature, the above mentioned oil solutions kept clear. According to the HPLC measurement, the residue contents were all no less than 98.3% of the initial value, indicating that no degradation occurred to the drugs. After 6 months of storage at temperature of 2-4°C, the above mentioned oil solutions kept clear. According to the HPLC measurement, the residue contents were all no less than 97.7% of the initial value, indicating a stable quality.

The above mentioned oil solutions contain emulsifier. In the case of adding 50 times of water, it can be emulsified after being magnetic stirred for 3 minutes, with the average particle size being ≤1μm after emulsification.

**Example 11: Oil solutions containing insulin/phospholipid complex (containing emulsifier)**

10g medium chain triglyceride (medium chain oil) was weighed, followed by addition of Cremphor RH40 in an amount of 1g, 2g and 4g respectively. After homogeneously mixing, 3 oil phases with different emulsifier ratio were prepared.

| Formulation Composition | Oil Phase 1 | Oil Phase 2 | Oil Phase 3 |
|---|---|---|---|
| Medium Chain Triglyceride | 10g | 10g | 10g |
| Cremphor RH40 | 1g | 2g | 4g |

3 portions of complex obtained from examples 2 to 4 respectively (each with a drug/lipid ratio of 1:10, w/w) were individually weighed in a suitable amount, followed by addition of the above mentioned 3 oil phases with different emulsifier ratio respectively, each in an amount of 9.12g. Stirring was conducted to dissolve, obtaining oil solutions with a drug load of 8mg/g, followed by filtering.

| Formulation Composition | Formulation 1 | Formulation 2 | Formulation 3 |
|---|---|---|---|
| Complex in an Amount of 880mg | Example 2 | Example 3 | Example 4 |
| Oil phase in an Amount of 9.12g | Oil Phase 1 | Oil Phase 2 | Oil Phase 3 |

After 24 hours of storage at room temperature, the above mentioned oil solutions kept clear. According to the HPLC measurement, the residue contents were all no less than 98.6% of the initial value, indicating that no degradation occurred to the drug. After 6 months of storage at temperature of 2-4°C, the above mentioned oil solutions kept clear. According to the HPLC measurement, the residue contents were all no less than 99.2% of the initial value, indicating a stable quality.

The above mentioned oil solutions contain emulsifier. In the case of adding 50 times of water, it can be emulsified after being magnetic stirred for 3 minutes, with the average particle size being ≤1μm after emulsification.

**Example 12: Oil solutions containing insulin/phospholipid complex (containing emulsifier and co-emulsifier)**

4 portions of complex obtained from examples 1 to 4 respectively (each with a drug/lipid ratio of 1:10, w/w) were individually weighed in a suitable amount, followed by addition of oil, emulsifier and co-emulsifier according to the table below. Stirring was conducted to dissolve, obtaining concentrated solutions of self-microemulsion with a drug load of 10mg/g.

| Formulation Composition | Formulation 1 | Formulation 2 | Formulation 3 | Formulation 4 |
|---|---|---|---|---|
| Complex 1100mg | Example 1 | Example 2 | Example 3 | Example 4 |
| Cremphor RH40 | 4g | 4g | 4g | 4g |
| Propanediol | 5g | / | / | / |
| Transcutol P | / | 4.5g | 4g | 3.5g |

Solutions of medium chain oil/long chain oil (1:1) was added to sum up 10g.

After 24 hours of storage at room temperature, the above mentioned 4 oil solutions kept clear. According to the HPLC measurement, the residue contents were all no less than 98.3% of the initial value. After 6 months of storage at temperature of -4°C, the above mentioned oil solutions kept clear. According to the HPLC measurement, the residue contents were all no less than 97.8% of the initial value, indicating a stable quality.

The above mentioned 4 oil solutions contain emulsifier and co-emulsifier. In the case of adding 5-500 times of water, diluted hydrochloric acid or buffer with pH6.8, it can be emulsified instantaneously. According to measurement on laser particle analyzer, the average particle size is in the range of 20~50nm after emulsification.

**Example 13: Vesicle solutions containing insulin/phospholipid complex**

Each of complexes obtained from examples 1-3 (each with a drug/lipid ratio of 1:10, w/w) was weighed in a suitable amount into a round-bottom flask, followed by addition of a suitable amount of free phospholipid (the amount of free phospholipid is equivalent to the amount of phospholipid contained in the complex) and addition of 20ml dichloromethane. The complex and the phospholipid dissolved and the insulin concentration was controlled to be 1mg/mL-10mg/mL. Rotary evaporation was conducted in vacuum in a water bath at 37°C. After formation of film through volatilization, 10mL PBS solution was added to hydrate for 1 hour so as to form a multicellular vesicle, followed by ultrasonic disruption or high pressure homogenization so as to obtain monocellular vesicle with a particle size of about 50nm.

| Formulation Composition | Formulation 1 | Formulation 2 | Formulation 3 |
|---|---|---|---|
| Complex (Examples 1-3) | Example 1 110mg | Example 2 220mg | Example 3 550mg |
| Free Phospholipid | 100mg | 200mg | 500mg |

**Example 14: Vesicle solutions containing insulin/phospholipid complex**

Each of complexes obtained from examples 1-8 (each with a drug/lipid ratio of 1:10, w/w) was weighed in a suitable amount into a round-bottom flask, followed by addition of a suitable amount of free phospholipid (the amount of free phospholipid is equivalent to the amount of phospholipid contained in the complex) and addition of a suitable amount of one or more surfactants such as Tween20 or Span60. Dichloromethane was added such that the complex and the phospholipid dissolved and the insulin concentration was controlled to be 1mg/mL-10mg/mL. Rotary evaporation was conducted in vacuum in a water bath at 37°C. After formation of film through volatilization, 10mL PBS solution was added to hydrate for 1 hour so as to form multicellular vesicle, followed by ultrasonic disruption or high pressure homogenization so as to obtain monocellular vesicle with a particle size of about 50nm.

| Formulation Composition | Formulation 1 | Formulation 2 | Formulation 3 |
|---|---|---|---|
| Complex (Examples 1-3) | Example 1 110mg | Example 2 220mg | Example 3 550mg |
| Free Phospholipid | 100mg | 200mg | 500mg |
| Tween20 | 200mg | 400mg | 600mg |

| Formulation Composition | Formulation 1 | Formulation 2 | Formulation 3 |
|---|---|---|---|
| Complex (Examples 1-3) | Example 1 110mg | Example 2 220mg | Example 3 550mg |
| Free Phospholipid | 100mg | 200mg | 500mg |
| Span60 | 200mg | 400mg | 600mg |

**Example 15: Vesicle powders containing insulin/phospholipid complex**

The vesicle powders were prepared by freeze drying the vesicle solutions obtained from examples 13 and 14.

**Test Examples**

**Test Example 1: Stability of oil solutions containing insulin complex in gastrointestinal bio-enzyme environment**

Test samples: Insulin solution (INS)
Suspension prepared by dissolving insulin complex (Phytosome) in water
Oil solutions obtained from example 8 to example 12
Test samples were placed in artificial gastric juice containing 1% (weight/volume, g/ml) of pepsase, followed by incubation in a water bath at 37 °C with vortex blending. Sampling was conducted in an amount of 0.5 ml after 1 min, 5 min, 30 min and 60 min respectively, followed by addition of 0.1 ml cold Tris solution (6.07g Tris reagent, adding water to sum up to 500 ml) with vortex blending. Centrifugation was conducted at 10000rpm for 5 min. The supernatant fluid was separated for HPLC measurement to determine the insulin residue percentage. The results were as follows:

| Samples | 1 min | 5 min | 30 min | 60 min |
|---|---|---|---|---|
| Insulin Solution (INS) | 0.56% | not detectable | not detectable | not detectable |
| Insulin Complex (Phytosome) | 50.4% | 35.4% | 28.4% | 12.4% |
| Oil Solutions Obtained from Example 8 - Example 12 | All above 87.2% | All above 63.9% | All above 43.7% | All above 35.2% |

Test Example 2: Stability of vesicle solutions containing insulin complex in gastrointestinal bio-enzyme environment

Test samples: insulin solution (INS)
common insulin vesicle (insulin instead of insulin complex, prepared as above)
new vesicle containing complex obtained from example 13
Test samples were placed in artificial gastric juice containing 1% (weight/volume, g/ml) of pepsase, followed by incubation in a water bath at 37 °C with vortex blending. Sampling was conducted in an amount of 0.5 ml after 1 min, 5 min, 30 min and 60 min respectively, followed by addition of 0.1 ml cold Tris solution (6.07g Tris reagent, adding water to sum up to 500 ml) with vortex blending. Centrifugation was conducted at 10000rpm for 5 min. The supernatant fluid was separated for HPLC measurement to determine the insulin residue percentage. The results were as follows:

| Sample | 1 min | 5 min | 30 min | 60 min |
|---|---|---|---|---|
| Insulin Solution (INS) | 0.76% | not detectable | not detectable | not detectable |
| Common Insulin Vesicle | 53.5-56.1% | 33.2-37.4% | 22.1-28.6% | 11.5-14.7% |
| Vesicle Obtained from Example 13 | 82.2-85.2% | 68.3-72.9% | 43.2-45.7% | 30.8-33.2% |

**Test Example 3: Caco-2 Cell permeability of vesicle solution containing insulin complex**

Test samples: Insulin solution (INS)
Common insulin vesicle (insulin instead of insulin complex, prepared as above)
New vesicle containing complex obtained from example 13

0.5 mL insulin solution, common insulin vesicle and insulin phospholipid complex vesicle (Example 13) with the same concentration were measured accurately and placed on the 12WL Caco-2 cells, with 1.5 mL HBSS solution being used below the cells as receptive medium, followed by incubation in air bath at 37 °C. Sampling was conducted in an amount of 200 $\mu$l after 30 min, 60 min, 120 min, 180 min and 240 min respectively. HPLC measurement was conducted to determine the accumulated permeability. The results were as follows:

| Samples | Accumulated Permeability (%) |
|---|---|
| Insulin Solution (INS) | 1.65 |
| Common Insulin Vesicle | 8.70 |
| Vesicle Obtained from Example 13 | No less than 17.52 |

**Test Example 4: Hypoglycemic effect of the oil solution and new vesicle containing insulin complex**

Normal male rats having weight of 200±20g were fasted for 12h (overnight), then subjected to intraperitoneal injection of 10 mg/ml streptozocin-trisodium citrate buffer (pH of about 4.5) in a dose of 60mg/kg. After one week stabilization, rats with blood sugar level of more than 16.7 mmol / 1 were determined as diabetic model.

Rats were fasted overnight before test without deprivation of water, and allowed to drink freely during experimentation.35 rats were randomly divided into 5 groups to be administrated according to the following regime. The blood sugar was determined by glucometer using tail blood collected at different time points.

The first group: blank control,

The second group: common insulin vesicle, orally administrated in a dose of 70IU/kg

The third group: oil solution of formulation 1 of example 9 prepared by medium chain oil, orally administrated in a dose of 70IU/kg

The fourth group: new nanometer vesicle prepared in example 13, orally administrated in a dose of 70IU/kg.

Blood sugar percentages of each animal at each time point were calculated so as to obtain a hypoglycemic effect curve with hypoglycemic percentage as Y-axis and time as X-axis. See Fig. 1.

## Claims

1. A process for the preparation of an insulin-lipid complex, comprising:

   - dissolving an insulin in methanol containing trifluoroacetic acid to form a clear insulin solution;
   - dissolving a lipid material in dichloromethane to form a clear lipid solution;
   - homogeneously mixing of the insulin solution and the lipid solution; and
   - conducting rotary evaporation under reduced pressure in a water bath, evacuating by a water pump, and drying, wherein the insulin is recombinant human insulin, the lipid material is a natural phospholipid or a salt thereof, the mass ratio of the insulin to the lipid material is between 1:5 and 1:10, the concentration of trifluoroacetic acid in methanol is 0.05-0.1%, and the amount of dichloromethane is 4-6 times of that of methanol.

2. The process according to claim 1, wherein the natural phospholipid is selected from egg yolk phospholipid or soybean phospholipid.

## Patentansprüche

1. Verfahren zur Herstellung eines Insulin-Lipid-Komplexes, umfassend: Auflösen eines Insulins in Methanol, das Trifluoressigsäure enthält, um eine klare Insulinlösung zu bilden; Auflösen eines Lipidmaterials in Dichlormethan, um eine klare Lipidlösung zu bilden; homogenes Mischen der Insulinlösung und der Lipidlösung; und Durchführen einer Rotationsverdampfung unter reduziertem Druck in einem Wasserbad, Evakuieren durch eine Wasserpumpe und Trocknen, wobei das Insulin rekombinantes menschliches Insulin ist, das Lipidmaterial ein natürliches Phospholipid oder ein Salz davon ist, das Massenverhältnis des Insulins zu dem Lipidmaterial zwischen 1:5 und 1:10 ist, die Konzentration von Trifluoressigsäure in Methanol 0,05-0,10 % ist und die Menge an Dichlormethan das 4-6-fache der von Methanol ist.

**2.** Verfahren nach Anspruch 1, wobei das natürliche Phospholipid ausgewählt ist aus Eigelb-Phospholipid oder Soja-Phospholipid.

**Revendications**

**1.** Processus destiné à la préparation d'un complexe insuline-lipide, comprenant : la dissolution d'une insuline dans du méthanol contenant de l'acide trifluoroacétique afin de former une solution d'insuline limpide ;
la dissolution d'un matériau à base de lipide dans du dichlorométhane afin de former une solution d'insuline limpide ;
le mélange homogène de la solution d'insuline et de la solution de lipide ; et réaliser l'évaporation rotative sous pression réduite dans un bain d'eau, l'évacuation au moyen d'une pompe à eau, et le séchage, l'insuline étant de l'insuline humaine recombinante, le matériau à base de lipide étant un phospholipide naturel ou un sel de celui-ci, le rapport de masse de l'insuline au matériau à base de lipide étant compris entre 1:5 et 1:10, la concentration de l'acide trifluoroacétique dans le méthanol étant comprise entre 0,05 et 0,1%, et la quantité de dichlorométhane étant 4 à 6 fois celle du méthanol.

**2.** Processus selon la revendication 1, dans lequel le phospholipide naturel est choisi parmi le phospholipide de jaune d'oeuf ou le phospholipide de soja.

# Figures

Legend:

The first group        blank control
The second group        vesica
The third group        oily solution
The fourth group the nano-vesicle

Time (h)

Fig.1

liposome

Phospholipid molecule
water-soluble durgs
lipid-soluble drugs

phospholipid complex

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9011780 A
- DE 3240177
- WO 2010060667 A1
- CN 01140047
- CN 97196069
- NO 01140047
- WO 97196069 A
- WO 01140047 A

### Non-patent literature cited in the description

- **CUI et al.** *Journal of Controlled Release,* vol. 114 (2), 242-250
- **SEMALTY et al.** *Expert Opinion on Drug Delivery,* vol. 6 (6), 599-612
- **PERRY et al.** *Biochemical Journal,* 1971, vol. 125 (1), 179-187
- **ZHOU et al.** *Journal of Peptide Science,* vol. 18 (9), 541-548